(19) Europäisches Patentamt
European Patent Office
Office européen des brevets

(11) Veröffentlichungsnummer: **0 472 078 A2**

# (12) EUROPÄISCHE PATENTANMELDUNG

(21) Anmeldenummer: **91113483.1**

(22) Anmeldetag: **12.08.91**

(51) Int. Cl.[5]: **C07K 5/02**, C07F 9/572, C07F 9/59, A61K 37/64, A61K 31/675

(30) Priorität: **23.08.90 DE 4026614**

(43) Veröffentlichungstag der Anmeldung: **26.02.92 Patentblatt 92/09**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Häbich, Dieter, Dr.**
**Krummacherstrasse 82**
**W-5600 Wuppertal(DE)**
Erfinder: **Hansen, Jutta, Dr.**
**Claudiusweg 9**
**W-5600 Wuppertal(DE)**
Erfinder: **Paessens, Arnold, Dr.**
**Stresemannstrasse 51**
**W-5657 Haan(DE)**

## (54) Phosphonopyrrolidin- und piperidin-haltige Pseudopeptide vom Statintyp, Verfahren zu ihrer Herstellung und ihre Verwendung als Arzneimittel gegen Retroviren.

(57) Die Erfindung betrifft neue Phosphonopyrrolidin- und -piperidin-haltige Pseudopeptide, Verfahren zu ihrer Herstellung und ihre Verwendung als Arzneimittel, insbesondere als antivirale Mittel in der Human- und Tiermedizin.

EP 0 472 078 A2

Die Erfindung betrifft neue Phosphonopyrrolidin- und piperidin-haltige Pseudopeptide, Verfahren zu ihrer Herstellung und ihre Verwendung als Arzneimittel, insbesondere als antivirale Mittel in der Human- und Tiermedizin.

Es wurde schon versucht, Pseudopeptide, die teilweise auch renininhibitorisch wirksam sind, bei der Bekämpfung von AIDS einzusetzen [vgl. GB A2 203 740; EP 337 714; EP 342 541; EP 346 847 und EP 352 000; EP 354 522; EP 357 332; EP 356 223].

Die vorliegende Erfindung betrifft neue phosphonopyrrolidin- und piperidin-haltige Pseudopeptide der allgemeinen Formel (I)

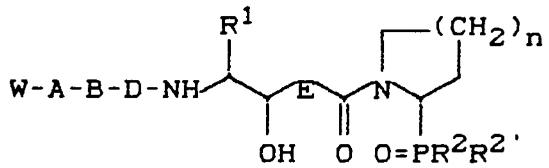

(I)

in welcher

W

- für Wasserstoff oder für eine typische Aminoschutzgruppe steht, oder
- für geradkettiges oder verzweigtes Alkyl oder Alkenyl mit jeweils bis zu 6 Kohlenstoffatomen steht, die gegebenenfalls durch Aryl mit 6 bis 10 Kohlenstoffatomen substituiert sind, oder
- für eine Gruppe der Formel $R^3$-CO-, $R^5R^4$N-CO- oder $R^6$-$SO_2$- steht,

  worin

  $R^3$

  - Wasserstoff, Trifluormethyl oder geradkettiges oder verzweigtes Alkoxy mit bis zu 8 Kohlenstoffatomen oder Alkyl mit bis zu 18 Kohlenstoffatomen bedeutet, das gegebenenfalls bis zu 2-fach durch Aryl mit 6 bis 10 Kohlenstoffatomen oder Pyridyl substituiert ist, oder
  - Aryl mit 6 bis 10 Kohlenstoffatomen bedeutet, das gegebenenfalls durch Halogen, Trifluormethyl, Trifluormethoxy oder durch geradkettiges oder verzweigtes Alkyl mit bis zu 8 Kohlenstoffatomen substituiert ist,
  - Cycloalkyl mit 3 bis 7 Kohlenstoffatomen bedeutet, oder
  - Chinolyl, Indolyl, Pyridyl, Morpholino oder Piperazinyl bedeutet, oder
  - einen Rest der Formel

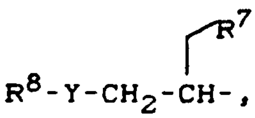, 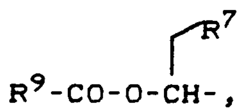, 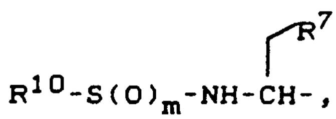,

$T-NH-(CH_2)_p-$ , 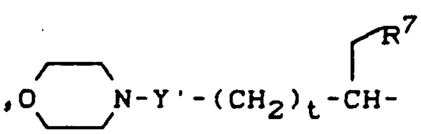

oder 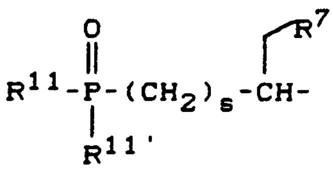

bedeutet,

worin

$R^7$ - Phenyl oder Naphthyl bedeutet,

$R^8$, $R^9$ und $R^{10}$ gleich oder verschieden sind und geradkettiges oder verzweigtes

Alkyl mit bis zu 17 Kohlenstoffatomen bedeuten, das gegebenenfalls durch Phenyl oder Naphthyl substituiert ist, oder Aryl mit 6 bis 10 Kohlenstoffatomen bedeuten, welches seinerseits durch Alkyl mit bis zu 4 Kohlenstoffatomen substituiert ist,

m - eine Zahl 0, 1 oder 2 bedeutet,

T - Morpholino oder Cyclohexyl bedeutet,

p - eine Zahl 1, 2 oder 3 bedeutet,

Y und Y' gleich oder verschieden sind und die CO- oder $SO_2$-Gruppe bedeuten,

t - eine Zahl 0 oder 1 bedeutet,

$R^{11}$ und $R^{11'}$ gleich oder verschieden sind und Hydroxy oder Alkoxy mit bis zu 8 Kohlenstoffatomen bedeuten,

s - eine Zahl 1 oder 2 bedeutet,

$R^4$ und $R^5$ gleich oder verschieden sind und

- Wasserstoff oder
- Aryl mit 6 bis 10 Kohlenstoffatomen bedeuten, das gegebenenfalls durch geradkettiges oder verzweigtes Alkyl mit bis zu 6 Kohlenstoffatomen oder Halogen substituiert ist, oder
- Cycloalkyl mit 3 bis 7 Kohlenstoffatomen bedeuten, oder
- geradkettiges oder verzweigtes Alkyl mit bis zu 18 Kohlenstoffatomen bedeuten,

$R^6$ - geradkettiges oder verzweigtes Alkyl mit bis zu 4 Kohlenstoffatomen oder Phenyl bedeutet, das seinerseits durch Methyl substituiert sein kann,

A, B und D gleich oder verschieden sind und

- für eine direkte Bindung oder
- für einen Rest der Formel

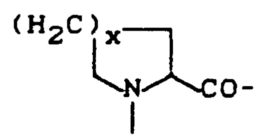 oder 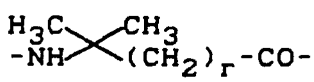

stehen,

worin

x - die Zahl 1 oder 2 bedeutet

und

r - die Zahl 0 oder 1 bedeutet,

oder

- für eine Gruppe der Formel

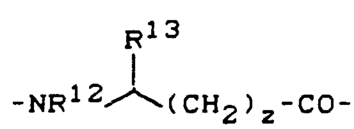

stehen

worin

z - die Zahl 0 oder 1 bedeutet,

$R^{12}$ Wasserstoff oder geradkettiges oder verzweigtes Alkyl mit bis zu 4 Kohlenstoffatomen bedeutet,

$R^{13}$ Wasserstoff, Cycloalkyl mit 3 bis 8 Kohlenstoffatomen oder Aryl mit 6 bis 10 Kohlenstoffatomen bedeutet, oder

- geradkettiges oder verzweigtes Alkyl mit bis zu 8 Kohlenstoffatomen bedeutet, das gegebenenfalls durch Methylthio, Hydroxy, Mercapto, Guanidyl oder durch eine Gruppe der Formel $-NR^{14}R^{15}$ oder $R^{16}$-OC-substituiert ist,

worin

$R^{14}$ und $R^{15}$ gleich oder verschieden sind und Wasserstoff, geradkettiges oder

verzweigtes Alkyl mit bis zu 8 Kohlenstoffatomen oder Phenyl bedeuten
und
$R^{16}$ Hydroxy, Benzyloxy, Alkoxy mit bis zu 6 Kohlenstoffatomen oder die oben aufgeführte Gruppe $-NR^{14}R^{15}$ bedeutet,
oder das gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{14}R^{15}$ substituiert ist,
worin
$R^{14}$ und $R^{15}$ die oben angegebene Bedeutung haben,
oder das gegebenenfalls durch einen 5- oder 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^1$ - für geradkettiges oder verzweigtes Alkyl oder Alkenyl mit bis zu 10 Kohlenstoffatomen steht, die gegebenenfalls durch Cycloalkyl mit 3 bis 8 Kohlenstoffatomen oder Aryl mit 6 bis 10 Kohlenstoffatomen substituiert sind, das seinerseits durch Halogen, Nitro, Hydroxy, Amino oder geradkettiges oder verzweigtes Alkoxy mit bis zu 4 Kohlenstoffatomen substituiert sein kann,

n - für die Zahl 1 oder 2 steht,

$R^2$ und $R^{2'}$ gleich oder verschieden sind und
- für Hydroxy oder Alkoxy mit bis zu 8 Kohlenstoffatomen stehen,
und

E - für einen Rest der Formel

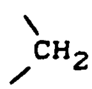, 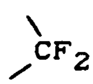 oder

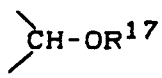

steht,
worin
$R^{17}$ - Wasserstoff oder geradkettiges oder verzweigtes Alkyl mit bis zu 4 Kohlenstoffatomen bedeutet,

und deren physiologisch unbedenklichen Salze.

Die erfindungsgemäßen Verbindungen der allgemeinen Formel (I), haben mehrere asymmetrische Kohlenstoffatome.

Sie können unabhängig voneinander in der D- oder der L-Form vorliegen. Die Erfindung umfaßt die optischen Antipoden ebenso wie die Isomerengemische oder Racemate. Bevorzugt liegen die Gruppen A, B und D unabhängig voneinander in der optisch reinen, bevorzugt in der L-Form vor.

Der Rest der allgemeinen Formel (VIII)

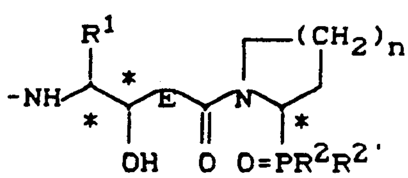

(VIII)

besitzt in Abhängigkeit von der Bedeutung des Restes E 3 oder 4 asymmetrische Kohlenstoffatome (*), die unabhängig voneinander in der R- oder S-Konfiguration vorliegen können.

Aminoschutzgruppe im Rahmen der Erfindung sind die üblichen in der Peptid-Chemie verwendeten

Aminoschutzgruppen.
Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Allyloxycarbonyl, Vinyloxycarbonyl, 2-Nitrobenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen.

Die erfindungsgemäßen Verbindungen der allgemeinen Formel (I) können in Form ihrer Salze vorliegen. Dies können Salze mit anorganischen oder organischen Säuren oder Basen sein.

Bevorzugt sind Verbindungen der allgemeinen Formel (I),
in welcher
W
- für Wasserstoff, tert.Butyloxycarbonyl (BOC), 9-Fluorenylmethyloxycarbonyl (Fmoc) oder Benzyloxycarbonyl steht, oder
- für geradkettiges oder verzweigtes Alkyl oder Alkenyl mit jeweils bis zu 4 Kohlenstoffatomen steht, die gegebenenfalls durch Phenyl substituiert sind, oder
- für eine Gruppe der Formel $R^3$-CO-, $R^5R^4$N-CO- oder $R^6$-$SO_2$- steht,
  worin
  $R^3$
  - Wasserstoff, Trifluormethyl oder geradkettiges oder verzweigtes Alkoxy mit bis zu 4 Kohlenstoffatomen oder Alkyl mit bis zu 16 Kohlenstoffatomen bedeutet, das gegebenenfalls bis zu 2-fach durch Phenyl, Naphthyl oder Pyridyl substituiert ist, oder
  - Phenyl oder Naphthyl bedeutet, die gegebenenfalls durch Fluor, Chlor, Trifluormethyl, Trifluormethoxy oder durch geradkettiges oder verzweigtes Alkyl mit bis zu 6 Kohlenstoffatomen substituiert sind,
  - Cyclopropyl, Cyclopentyl, Cyclohexyl, Chinolyl, Indolyl, Pyridyl, Morpholino oder Piperazinyl bedeutet, oder
  - einen Rest der Formel

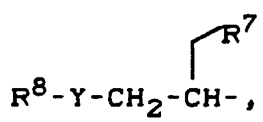 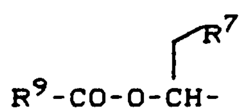 oder

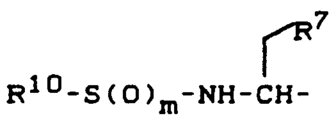

bedeutet,
worin
Y - die CO- oder $SO_2$-Gruppe bedeutet,
$R^7$ - Phenyl oder Naphthyl bedeutet,
$R^8$, $R^9$ und $R^{10}$ gleich oder verschieden sind und geradkettiges oder verzweigtes Alkyl mit bis zu 15 Kohlenstoffatomen, Tolyl, Phenyl oder Naphthyl bedeuten,
m - eine Zahl 1 oder 2 bedeutet,

$R^4$ und $R^5$ gleich oder verschieden sind und
- Wasserstoff oder
- Phenyl oder Naphthyl bedeuten, die gegebenenfalls durch geradkettiges oder verzweigtes Alkyl mit bis zu 4 Kohlenstoffatomen, Fluor oder Chlor substituiert sind,
- Cyclopropyl, Cyclopentyl oder Cyclohexyl bedeuten, oder

- geradkettiges oder verzweigtes Alkyl mit bis zu 16 Kohlenstoffatomen bedeuten,
$R^6$ - Phenyl bedeutet, das durch Methyl substituiert ist,

A, B und D gleich oder verschieden sind und
- für eine direkte Bindung oder
- für Prolin stehen, oder
- für einen Rest der Formel

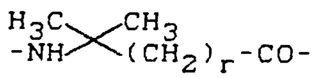

stehen,
worin
r die Zahl 0 oder 1 bedeutet
- für eine Gruppe der Formel

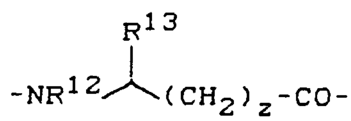

stehen
worin
z - die Zahl 0 oder 1 bedeutet,
$R^{12}$ - Wasserstoff, Methyl oder Ethyl bedeutet,
$R^{13}$ - Wasserstoff, Cyclopentyl, Cyclohexyl oder Phenyl bedeutet,
- oder geradkettiges oder verzweigtes Alkyl mit bis zu 6 Kohlenstoffatomen bedeutet, das gegebenenfalls durch Methylthio, Hydroxy, Mercapto, Guanidyl, Amino, Carboxy oder $H_2N$-CO-substituiert sein kann,
oder durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,
oder durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^1$ - für geradkettiges oder verzweigtes Alkyl oder Alkenyl mit bis zu 8 Kohlenstoffatomen steht, die gegebenenfalls durch Cyclopropyl, Cyclobutyl, Cyclopentyl, Cyclohexyl, Cycloheptyl oder Phenyl substituiert sind, das seinerseits durch Fluor, Chlor, Brom, Nitro, Hydroxy oder Amino substituiert ist,

n - für die Zahl 1 oder 2 steht,

$R^2$ und $R^{2'}$ gleich oder verschieden sind und
- für Hydroxy oder Alkoxy mit bis zu 6 Kohlenstoffatomen stehen,
und

E - für einen Rest der Formel $CH_2$, $CF_2$ oder CH-$OR^{17}$ steht,
worin
$R^{17}$ - Wasserstoff oder geradkettiges oder verzweigtes Alkyl mit bis zu 4 Kohlenstoffatomen steht

und deren physiologisch unbedenklichen Salze.

Besonders bevorzugt sind Verbindungen der allgemeinen Formel (I),
in welcher

W
- für Wasserstoff, tert.Butyloxycarbonyl (BOC) oder Benzyloxycarbonyl steht, oder
- für Allyl oder Benzyl steht,
- für eine Gruppe der Formel $R^3$-CO-, $R^5R^4$N-CO- oder $R^6$-$SO_2$- steht,
worin
$R^3$

- Wasserstoff, Trifluormethyl oder geradkettiges oder verzweigtes Alkyl mit bis zu 14 Kohlenstoffatomen bedeutet, das gegebenenfalls bis zu 2-fach durch Phenyl, Naphthyl oder Pyridyl substituiert ist, oder
- Phenyl oder Naphthyl bedeutet, die gegebenenfalls durch Fluor, Chlor, Trifluormethyl, Trifluormethoxy oder durch geradkettiges oder verzweigtes Alkyl mit bis zu 4 Kohlenstoffatomen substituiert sind,
- Cyclopropyl, Cyclopentyl, Cyclohexyl, Chinolyl, Indolyl, Pyridyl, Morpholino oder Piperazinyl bedeutet, oder
- einen Rest der Formel

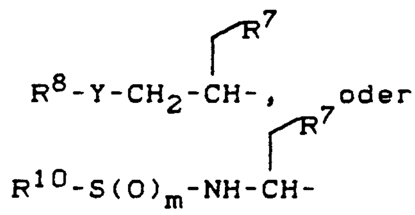
, oder

bedeutet,
worin
Y - die CO- oder $SO_2$-Gruppe bedeutet,
$R^7$ - Phenyl oder Naphthyl bedeutet,
$R^8$ und $R^{10}$ gleich oder verschieden sind und geradkettiges oder verzweigtes Alkyl mit bis zu 14 Kohlenstoffatomen, Tolyl, Phenyl oder Naphthyl bedeuten,
m - eine Zahl 1 oder 2 bedeutet,

$R^4$ und $R^5$ gleich oder verschieden sind und
- Wasserstoff oder
- Phenyl oder Naphthyl bedeuten, die gegebenenfalls durch Methyl, Fluor oder Chlor substituiert sind,
- Cyclopropyl, Cyclopentyl oder Cyclohexyl bedeuten, oder
- geradkettiges oder verzweigtes Alkyl mit bis zu 14 Kohlenstoffatomen bedeuten,

$R^6$ - Phenyl bedeutet, das durch Methyl substituiert ist,

A, B und D gleich oder verschieden sind und
- für eine direkte Bindung stehen, oder
- für Prolin stehen, oder
- für einen Rest der Formel

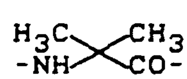

stehen,
- für eine Gruppe der Formel

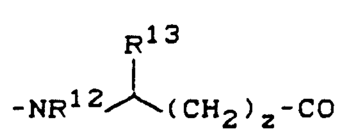

stehen,
worin
z - die Zahl 0 oder 1 bedeutet,
$R^{12}$- Wasserstoff oder Methyl bedeutet,
$R^{13}$
- Wasserstoff, Cyclopentyl oder Cyclohexyl bedeutet,

- oder geradkettiges oder verzweigtes Alkyl mit bis zu 4 Kohlenstoffatomen bedeutet, das gegebenenfalls durch Methylthio, Hydroxy, Mercapto, Guanidyl, Amino, Carboxy oder $H_2N$-CO-substituiert ist,
  oder durch Cyclohexyl, Naphthyl oder Phenyl substituiert ist, das seinerseits durch Hydroxy, Fluor, Chlor oder Alkoxy mit bis zu 4 Kohlenstoffatomen substituiert sein kann, oder
  durch Indolyl, Imidazolyl, Triazolyl, Pyridyl der Pyrazolyl substituiert ist, wobei die NH-Funktion gegebenenfalls durch Methyl, Benzyloxymethylen oder t-Butyloxycarbonyl (Boc) geschützt sind,

$R^1$ - für geradkettiges oder verzweigtes Alkyl mit bis zu 6 Kohlenstoffatomen steht, das gegebenenfalls durch Cyclopropyl, Cyclopentyl, Cyclohexyl oder Phenyl substituiert ist, das seinerseits durch Hydroxy substituiert sein kann,

n - für die Zahl 1 oder 2 steht,

$R^2$ und $R^{2'}$ gleich oder verschieden sind und
- für Hydroxy oder Alkoxy mit bis zu 4 Kohlenstoffatomen stehen,

und

E - für die

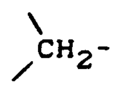

oder die

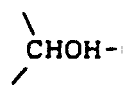

Gruppe steht

und deren physiologisch unbedenklichen Salze,

Außerdem wurde ein Verfahren zur Herstellung der erfindungsgemäßen Verbindungen der allgemeinen Formel (I),

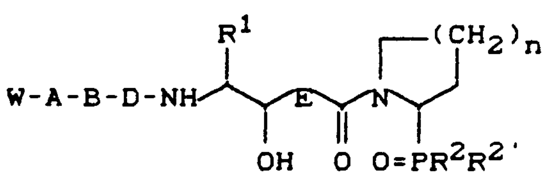

(I)

in welcher

W, A, B, D, $R^1$, $R^2$, $R^{2'}$, E und n die oben angegebene Bedeutung haben,

gefunden, dadurch gekennzeichnet, daß man

[A] Verbindungen der allgemeinen Formel (Ia)

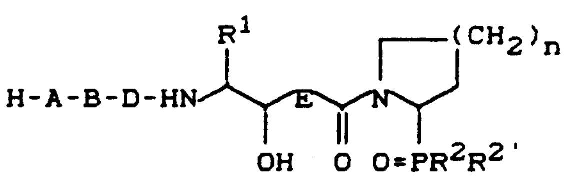

(Ia)

in welcher

A, B, D, $R^1$, $R^2$, $R^{2'}$, E und n die oben angegebene Bedeutung haben,

mit Verbindungen der allgemeinen Formeln (II) oder (III)

W-X (II)

$(W')_2O$ (III)

in welcher
W die oben angegebene Bedeutung hat
X für Halogen, vorzugsweise für Chlor steht,
und
W' für die Gruppe $CF_3CO$ oder $CH_3CO$ steht,
nach den in der Peptidchemie üblichen Bedingungen, in inerten Lösemitteln, in Anwesenheit einer Base umsetzt,
oder
[B] entweder Verbindungen der allgemeinen Formel (Ib)

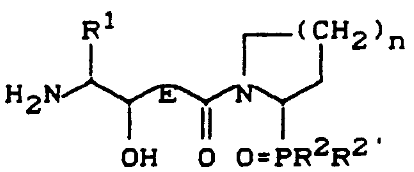

(Ib)

in welcher
$R^1$, $R^2$, $R^{2'}$, E und n die oben angegebene Bedeutung haben,
direkt mit Verbindungen der allgemeinen Formel (IV)

W-A'-B'-D'-OH (IV)

in welcher
W die oben angegebene Bedeutung hat,
A', B' oder D' die oben angegebene Bedeutung von A, B oder D haben und nicht gleichzeitig für eine Bindung stehen,
oder Verbindungen der allgemeinen Formel (Ic)

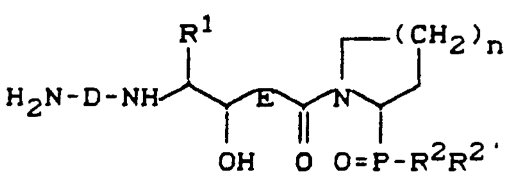

(Ic)

in welcher
D, $R^1$, $R^2$, $R^{2'}$, E und n die oben angegebene Bedeutung haben,
mit Verbindungen der allgemeinen Formel (IVa)

W-A'-B'-OH (IVa)

in welcher
W, A' und B' die oben angegebene Bedeutung haben,
unter Aktivierung der Carbonsäure, in inerten Lösemitteln, gegebenenfalls in Anwesenheit einer Base und eines Hilfsstoffes umsetzt,
oder
[C] Verbindungen der allgemeinen Formeln (V) und (VI)

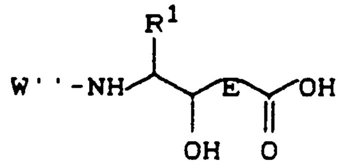

(V)

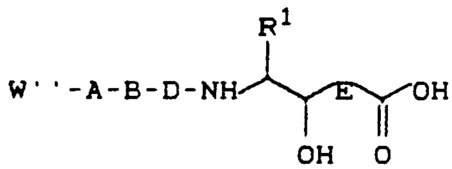

(VI)

in welcher
$R^1$, W, A, B, D und E die oben angegebene Bedeutung haben
und
W'' für eine Aminoschutzgruppe, vorzugsweise für BOC steht,
mit Verbindungen der allgemeinen Formel (VII)

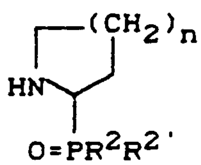

(VII)

in welcher
$R^2$, $R^{2'}$ und n die oben angegebene Bedeutung haben,
unter Aktivierung der Carbonsäuren, gegebenenfalls in Anwesenheit einer Base und eines Hilfsstoffes kondensiert und im Fall der Verbindungen der allgemeinen Formel (V) die Schutzgruppe W'' anschließend nach üblicher Methode abspaltet und gegebenenfalls mit den Verbindungen der allgemeinen Formeln (IV) oder (IVa) nach der unter Verfahren [B] beschriebenen Methode weiter umsetzt.

Die erfindungsgemäßen Verfahren können durch folgendes Formelschema beispielhaft erläutert werden:

[A]

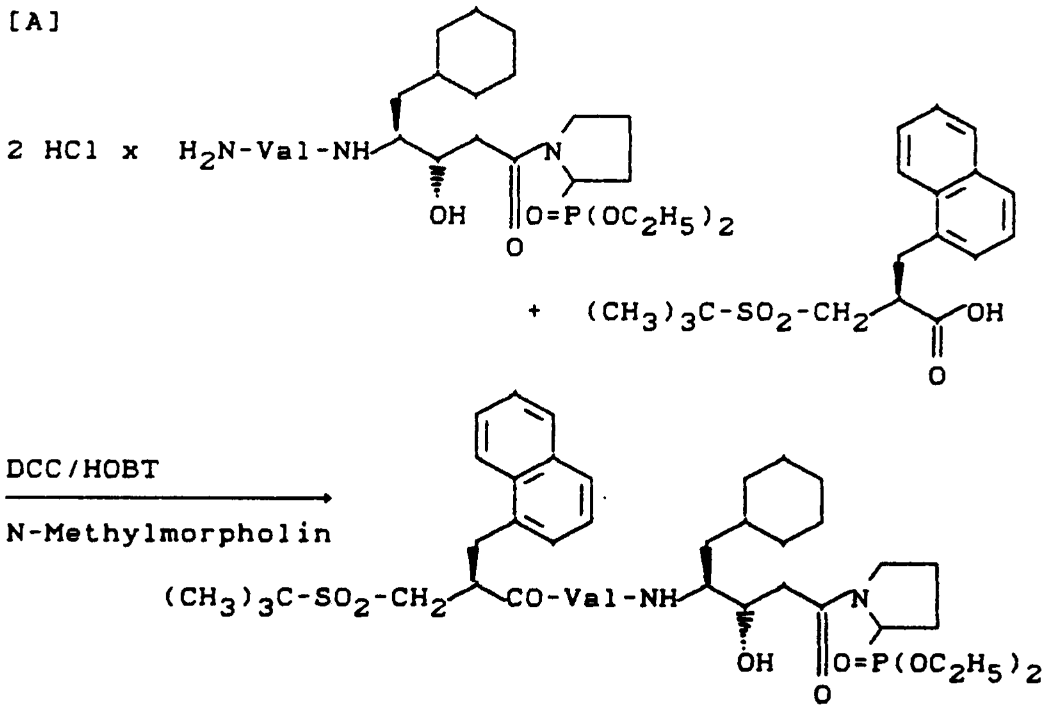

[B]

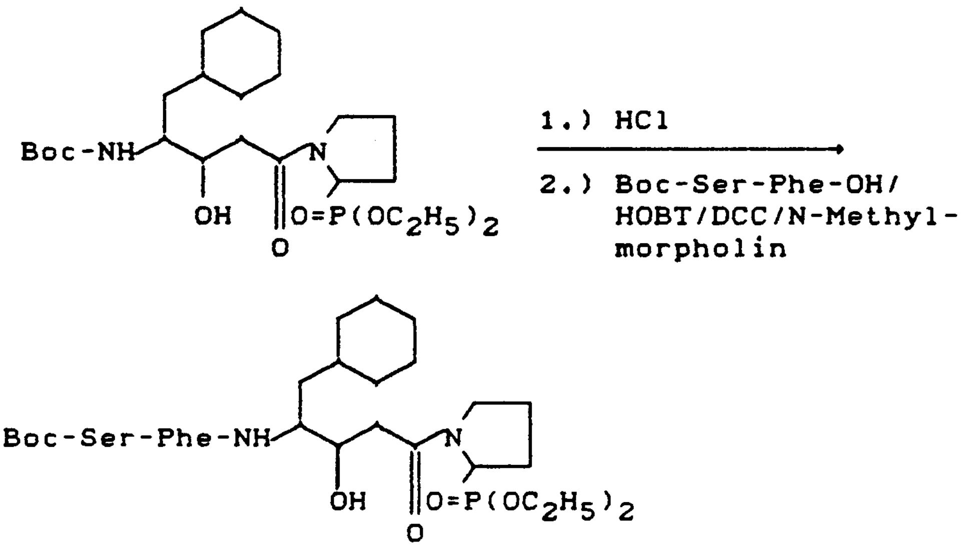


[C]

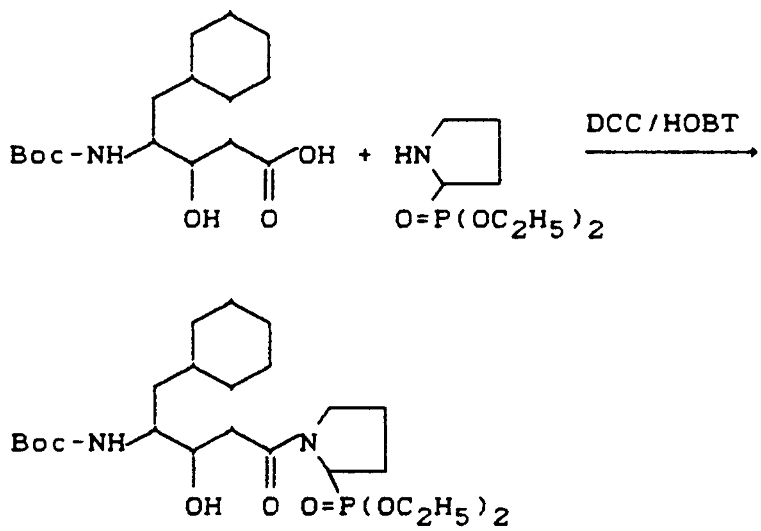


Als Lösemittel eignen sich für alle Verfahrensschritte die üblichen inerten Lösemittel, die sich unter den Reaktionsbedingungen nicht verändern. Hierzu gehören bevorzugt organische Lösemittel wie Ether z.B. Diethylether, Glykolmono-oder dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenwasserstoff, oder Dimethylsulfoxid, Dimethylformamid, Hexamethylphosphorsäuretriamid, Essigester, Pyridin, Triethylamin oder Picoline, Ebenso ist es möglich, Gemische der genannten Lösemittel zu verwenden, Besonders bevorzugt sind Dichlormethan, Chloroform, Dimethylformamid oder Tetrahydrofuran.

Die Verbindungen der allgemeinen Formeln (IV) und (IVa) sind an sich bekannt und können durch Umsetzung eines entsprechenden Bruchstückes, bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, mit einer Aminogruppe, gegebenenfalls in aktivierter Form, und durch Wiederholung dieses Vorgangs mit entsprechenden Bruchstücken hergestellt werden, anschließend können gegebenenfalls Schutzgruppen abspalten oder gegen andere Schutzgruppen austauscht werden [vgl. Houben-Weyl, Methoden der organischen Chemie, Synthese von Peptiden II, 4. Aufl, Bd. 15/1, 15/2, Georg Thieme Verlag, Stuttgart].

Als Hilfsstoffe für die Peptidkupplungen für die Einführung des Substituenten W (Formeln (II) und (III)) und des Phosphonopyrrolidin- und piperidinrings (Formel (VII)) werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfatoder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)-phosphoniumhexafluorophosphat.

Als Basen können sowohl bei den Peptidkupplungen als auch bei den oben aufgeführten Umsetzungen mit den Verbindungen der allgemeinen Formeln (II), (III) und (IV) Alkalicarbonate z.B. Natrium-oder Kaliumcarbonat oder -hydrogencarbonat, oder organische Basen wie Trialkylamine z.B. Triethylamin, N-Ethylmorpholin, N-Methylpiperidin oder N-Methylmorpholin eingesetzt werden. Bevorzugt ist N-Methylmorpholin.

Die Hilfsstoffe und Basen werden in einer Menge von 0,5 Mol bis 4 Mol, bevorzugt 1 bis 1,5 Mol, bezogen auf jeweils 1 Mol der Verbindungen der allgemeinen Formeln (II), (III), (IV), (IVa) und (VII) eingesetzt.

Die Peptidkupplungen, die Einführung des Substituenten W (II, III) sowie die Umsetzung mit Verbindungen der allgemeinen Formel (VII) werden in einem Temperaturbereich von 0 °C bis 100 °C, vorzugsweise bei 0 °C bis 30 °C und bei Normaldruck durchgeführt.

Die Reaktionen können sowohl bei Normaldruck als auch bei erhöhtem oder erniedrigtem Druck (beispielsweise 0,5 bis 5 bar), vorzugsweise bei Normaldruck durchgeführt werden.

Die Verbindungen der allgemeinen Formeln (Ia), (Ib) und (Ic) sind ebenfalls neu und können nach den unter den jeweiligen Verfahren [A] oder [B] oben aufgeführten Methoden hergestellt werden.

Die Verbindungen der allgemeinen Formeln (II) und (III) sind bekannt oder können nach üblicher Methode hergestellt werden.

Die Verbindungen der allgemeinen Formeln (V) und (VI) sind teilweise bekannt oder neu und können im letzteren Fall ausgehend von den entsprechenden Estern durch Verseifung nach üblicher Methode hergestellt werden [vgl. J. Med, Chem. 28, 1779 (1985), J.O.. 43, 3624 (1978), J. Med. Chem. 23, 27 (1980); J. Med. Chem. 29, 2080 (1986); EP 184 855; PCT WO 87 04349].

Die Verbindungen der allgemeinen Formel (VII) sind ebenfalls bekannt [n=1 vgl. US 4 186 268; Y. Nomura et al., Chem. Lett., 693 (1977); n = 2 vgl. V.A. Solodenko et al., Zh. Obshch. Khim. 57, 2392 (1987)].

Es wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T. Sukrow, S. and Mölling, K. (1988), EMBO Journal, Vol, 7, No. 6, pp. 1785-1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen $IC_{50}$-Werte beziehen sich auf die Substanzkonzentration, die unter den oben aufgeführten Testbedingungen eine 50%ige Hemmung der Protease-Aktivität bewirkt.

| Beispiel-Nr. | $IC_{50}$ (RP-HPLC) (M) |
|---|---|
| 5 | $10^{-5}$ |
| 9 (polar) | $10^{-4}$ |
| 11 | $10^{-4}$ |
| 12 | $10^{-4}$ |
| 14 | $5 \times 10^{-7}$ |
| 15d | $10^{-5}$ |
| 15e | $5 \times 10^{-7}$ |
| 15f | $10^{-6}$ |
| 15m | $5 \times 10^{-6}$ |
| 15n | $10^{-5}$ |
| 18 | $10^{-5}$ |
| 25 (polar) | $10^{-7}$ |
| 28 | $10^{-5}$ |

Außerdem zeigten die erfindungsgemäßen Verbindungen Wirkung in Lentivirus infizierten Zellkulturen. Dies konnte am Beispiel des HIV-Virus gezeigt werden.

Der HIV-Test wurde mit geringen Modifikationen nach der Methode von Pauwels et al, (Journal of Virological Methods 20 (1988) 309 - 321) durchgeführt.

Normale menschliche Blutlymphozyten (PBL's) wurden über Ficoll-Hypaque angereichert und in RPMI 1640, 20 % fötales Kälberserum mit Phythaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß $1 \times 10^5$ Zellen pro ml eingestellt waren. Die derart infizierten Zellen wurden zu $1 \times 10^4$ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfverbindungen in 2er Schritten $10^2$fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde, In der unbehandelten Viruskontrolle resultierten unter diesen Testbedingungen etwa 20 Syncytien, während die unbehandelte Zellkontrolle keine Syncytien aufwies.

Die IC-50 Werte wurden als die Konzentration der behandelten und infizierten Zellen ermittelt, bei der 50 % (ca. 10 Syncytien) der virusinduzierten Syncytien durch die Behandlung mit der erfindungsgemäßen Verbindung unterdrückt waren.

| Beispiel Nr. | IC-50 (µM) |
|---|---|
| 9 | 1,5 |
| 16 | 4,3 |

Es wurde gefunden, daß die erfindungsgemäßen Verbindungen HIV infizierter Zellen vor der virusinduzierten Zellzerstörung schützen.

Die erfindungsgemäßen Verbindungen stellen wertvolle Wirkstoffe zur Behandlung und Prophylaxe von Erkrankungen, hervorgerufen durch Retroviren, in der Human-und Tiermedizin dar.

Als Indikationsgebiete in der Humanmedizin können beispielsweise genannt werden:

1.) Die Behandlung oder Prophylaxe von menschlichen Retrovirusinfektionen.

2.) Für die Behandlung oder Prophylaxe von HIV I (Virus der humanen Immundefizienz; früher HTLV III/LAV genannt) und HIV II verursachten Erkrankungen (AIDS) und den damit assozierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV I-oder HTLV II-Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)

d) Zwoegerziokte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

## Anhang zum experimentellen Teil

### I. Liste der verwendeten Laufmittelgemische zur Chromatographie:

I Dichlormethan : Methanol
II Toluol : Ethylacetat
III Acetonitril : Wasser
IV Dichlormethan : Methanol : Ammoniak 9:1:0,1

### II. Aminosäuren

Im allgemeinen erfolgt die Bezeichnung der Konfiguration durch das Vorausstellen eines L bzw. D vor der Aminosäureabkürzung, im Fall des Racemats durch ein D,L-wobei zur Vereinfachung bei L-Aminosäuren die Konfigurationsbezeichnung unterbleiben kann und dann nur im Fall der D-Form bzw. des D,L-Gemisches explizierte Bezeichnung erfolgt.

| | |
|---|---|
| Ala | L-Alanin |
| Arg | L-Arginin |
| Asn | L-Asparagin |
| Asp | L-Asparaginsäure |
| Cys | L-Cystein |
| Gln | L-Glutamin |
| Glu | L-Glutaminsäure |
| Gly | L-Glycin |
| His | L-Histidin |

| | |
|---|---|
| Ile | L-Isoleucin |
| Leu | L-Leucin |
| Lys | L-Lysin |
| Met | L-Methionin |
| Pro | L- Prolin |
| Phe | L-Phenylalanin |
| Ser | L-Serin |
| Thr | L-Threonin |
| Trp | L-Tryptophan |
| Tyr | L-Tyrosin |
| Val | L-Valin |

III. Abkürzungen

| | |
|---|---|
| AIB | Aminoisobutyryl |
| Z | Benzyloxycarbonyl |
| BOC | tert.Butoxycarbonyl |
| CMCT | 1-Cyclohexyl-3-(2-morpholino-ethyl)-carbodiimid-metho-p-toluolsulfonat |
| DCC | Dicyclohexylcarbodiimid |
| DMF | Dimethylformamid |
| HOBT | 1-Hydroxybenzotriazol |
| Mir | Miristoyl |
| Ph | Phenyl |
| THF | Tetrahydrofuran |

Ausgangsverbindungen

Beispiel I

Boc-Aib-Phe-Val-$OCH_3$

Eine gerührte, auf 0° C gekühlte Lösung von 10,17 g (50,0 mmol) 2-(1,1-Dimethylethoxycarbonyl)-amino-2-methyl-propionsäure und 7,09 g (52,5 mmol) HOBT in 140 ml wasserfreiem Dichlormethan wurde mit 10.83 g (52,5 mmol) DCC versetzt. Das Kühlbad wurde entfernt, und man rührte 30 min bei Raumtemperatur. Danach wurde erneut auf 0° C gekühlt, man gab eine Lösung von 17,47 g (55,5 mmol) HCl x H-Phe-Val-$OCH_3$ und 13,75 ml (125,0 mmol) N-Methylmorpholin in 140 ml Dichlormethan zu und rührte 15 h im auftauenden Eisbad nach. Der ausgefallene Harnstoff wurde durch Filtration abgetrennt, das Filtrat wurde mit 2 x 100 g $NaHCO_3$-Lösung und 100 ml Wasser gewaschen und über $MgSO_4$ getrocknet. Nach Abdampfen des Lösemittels im Vakuum und Chromatographie des Rohprodukts an 270 g Kieselgel (Toluol: Ethylacetat 3:2) erhielt man 20,0 9 (86% der Theorie) der Titelverbindung als farblosen Schaum.
DC: $R_f$ = 0,38 (Toluol : Ethylacetat 1:1)
MS (DCI, $NH_3$): m/z = 464 $(M+H)^+$.
SF (MG): $C_{24}H_{37}N_3O_6$ (463,58)

Beispiel II

Boc-AIB-Phe-Val-OH

Eine Lösung von 13,0 g (28,0 mmol) der Verbindung aus Beispiel I in 10 ml THF wurde mit einer Lösung von 2,35 g (56,0 mmol) Lithiumhydroxidhydrat in 55 ml Wasser versetzt und noch 3 h bei 0° C gerührt. Danach goß man das Reaktionsgemisch in eine Gemisch aus 60 ml Wasser, 40 g Eis und 100 ml Ethylacetat und stellte durch Zugabe von 1 N Salzsäure pH 3 ein. Die organische Phase wurde abgetrennt, die Wasserphase mit 50 ml Ethylacetat extrahiert und die vereinigten organischen Extrakte wurden über Magnesiumsulfat getrocknet. Nach Abdampfen des Lösemittels im Vakuum und Behandeln des Rückstands mit 10 ml Ether und 30 ml n-Pentan erhielt man 10,3 g (82% der Theorie) der Titelverbindung als farblose Kristalle.
Schmp.: 157° C
HPLC-Reinheit: > 96%

DC: $R_f$ = 0,44 (Acetonitril:Wasser = 9:1)
MS (FAB): m/z = 450 $(M+H)^+$, 472 $(M+Na)^+$
SF (MG): $C_{23}H_{35}N_3O_6$ (449,55)

Beispiel III

Boc-AIB-Val-$OCH_3$

Wie für Beispiel 1 beschrieben erhielt man aus 10,17 g (50,0 mmol) 2-(1,1-Dimethylethoxycarbonyl)-amino-2-methylpropionsäure mit 9,19 g (55,5 mmol) HCl x H-Val-$OCH_3$ noch Chromatographie des Rohproduktes an 300 g Kieselgel (Toluol:Ethylacetat 1:1) 10,3 g (79% der Theorie) der Titelverbindung als farblose Kristalle.
Schmp.: 108° C
DC: $R_f$ = 0,43 (Toluol: Ethylacetat = 1:1)
MS (FAB): m/z = 317 $(M+H)^+$
SF (MG): $C_{15}H_{28}N_2O_5$ (316,40)

Beispiel IV

Boc-AIB-Val-OH

Wie für Beispiel II beschrieben erhielt man aus 5,0 g (15,8 mmol) der Verbindung aus Beispiel III 4,0 g (84% der Theorie der Titelverbindung als farbloses Pulver.
Schmp.: 162° C
DC: $R_f$ = 0,5 (Acetonitril : Wasser 9:1)
MS (FAB): m/z = 309 $(M+Li)^+$, 617 $(2M+2Li-H)^+$
SF (MG): $C_{14}H_{26}N_2O_5$ (302,38).

Herstellungsbeispiele

Beispiel 1

1-{(3S, 4S)-4-[(tert.Butoxycarbonyl)amino]-5-cyclohexyl-3-hydroxy-pentanoyl}-(2R,S)-2-(pyrrolidinyl)-phosphonsäure-diethylester

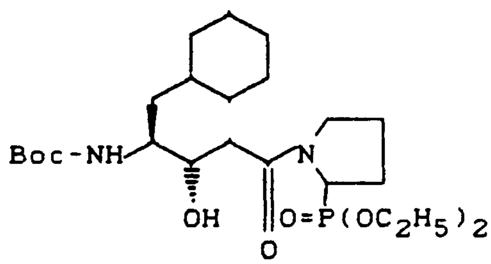

Eine gerührte Lösung von 16,57 g (52,53 mmol) N-(tert.-Butoxycarbonyl)-(3S, 4S)-4-amino-5-cyclohexyl-3-hydroxypentansäure [Boc-ACHPA; Boger et al., J Med. Chem. 28, 1779 (1985)] in 100 ml wasserfreiem DMF wurde bei 0° C mit 7,80 g (57,78 mmol) HOBT und 23,36 g (55,16 mmol) CMTC versetzt. Das Kühlbad wurde entfernt und man rührte 1 h bei Raumtemperatur nach. Danach wurde erneut auf 0° C gekühlt und man tropfte eine Lösung von 11,97 g (55,79 mmol) 2(R,S)-2-(Pyrrolidinyl)-phosphonsäurediethylester [US 4 186 268] und 14,00 ml (127,14 mmol) N-Methylmorpholin in 30 ml DMF zu. Das Kühlbad wurde entfernt und man rührte 17 h bei Raumtemperatur nach. Danach wurde die Reaktionsmischung im Vakuum eingeengt und der Rückstand zwischen 200 ml Wasser und 200 ml Ethylacetat verteilt. Die Wasserphase wurde mit 50 ml Ethylacetat extrahiert, die vereinigten Extrakte wurden mit 100 ml Wasser gewaschen und über $MgSO_4$ getrocknet. Nach Abdampfen des Lösemittels im Vakuum und Chromatographie des Rohproduktes an 800 g Kieselgel (Dichlormethan : Methanol 95:5) erhielt man 17,91 g (68% der Theorie) der Titelverbindung als Öl.

$R_f$ = 0,12 Laufmittelgemisch I (95:5)
MS (FAB): m/z = 505 $(M+H)^+$, 527 $(M+Na)^+$

Beispiel 2

1-[(3S, 4S)-4-Amino-5-cyclohexyl-3-hydroxypentanoyl]-(2R,S)-2-(pyrrolidinyl)-phosphonsäurediethylester Hydrochlorid

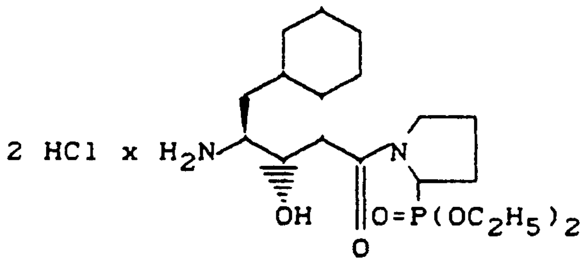

Eine Lösung von 17,95 g (35,57 mmol) der Verbindung aus Beispiel 1 in 178 ml einer 4 H Lösung gasförmigen Chlorwasserstoffs in wasserfreiem Dioxan wurde 30 min bei Raumtemperatur gerührt. Danach gab man 20 ml Toluol zu und engte im Vakuum ein, Dieser Vorgang wurde noch zweimal wiederholt, dann wurde der Rückstand mit wenig Ether verrieben, abgesaugt und im Hochvakuum über KOH getrocknet. Man erhielt 14,75 g (87% der Theorie) der Titelverbindung als hygroskopisches Pulver.
$R_f$ = 0,54, I (4:1)
MS (DCI, $NH_3$): m/z = 405 $(M+H)^+$

Beispiel 3

1-[(3S, 4S)-4-(tert.Butoxycarbonylasparaginyl)amino-5-cyclohexyl-3-hydroxypentanoyl]-(2R, S)-2-(pyrrolidinyl)-phosphonsäurediethylester

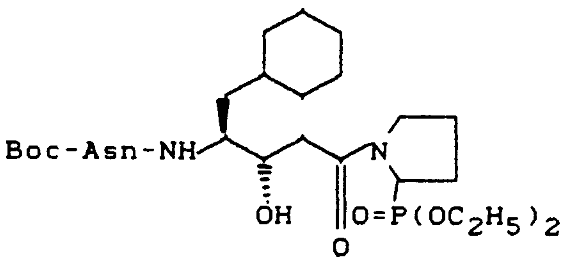

Eine gerührte Lösung von 3,48 g (15,00 mmol) tert.Butoxycarbonylasparagin und 2,23 g (16,50 mmol) HOBT in 30 ml wasserfreiem DMF wurde bei 0° C mit 6,67 g (15,75 mmol) CMTC versetzt und 2 h gerührt. Dazu tropfte man bei dieser Temperatur eine Lösung von 4,77 g (10,00 mmol) der Verbindung aus Beispiel 2 und 4,4 ml (40,00 mmol) N-Methylmorpholin in 15 ml DMF zu und rührte 17 h im auftauenden Eisbad nach. Danach wurde die Reaktionsmischung im Vakuum eingeengt und der Rückstand zwischen 50 ml Ethylacetat und 50 ml Wasser verteilt. Die Wasserphase wurde mit 30 ml Ethylacetat extrahiert und die vereinigten Extrakte wurden mit 50 ml Wasser gewaschen und über $MgSO_4$ getrocknet. Nach Abdampfen des Lösemittels im Vakuum und Chromatographie des Rückstandes an 80 g Kieselgel (Dichlormethan : Methanol 9:1) erhielt man 3,88 g (63% der Theorie) der Titelverbindung als Schaum.
$R_f$ = 0,30, I (95:5)
MS (FAB): m/z = 619 $(M+H)^+$, 641 $(M+Na)^+$

Wie für Beispiel 2 beschrieben, erhielt man aus den entsprechenden Boc-geschützten Verbindungen die folgenden Produkte (Tabelle 1)

Tabelle 1

2 HCl x H-A-B-D- 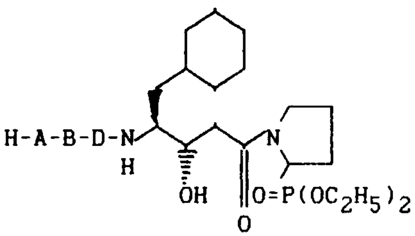

| Beispiel-Nr. | A-B | Ausbeute (%) | MS (FAB) m/z $(M+H)^+$ | $R_f$/Laufmittel- (Verhältnis) |
|---|---|---|---|---|
| 4 | Asn | 78 | 519[a] | 0,24, I (4:1) |
| 5 | Val | 48 | 504[a] | 0,36, I (9:1) |
| 6 | Phe[b] | 73 | 552 | 0,48, I (9:1) |
| 7 | Phe[c] | 89 | 552 | 0,30, I (9:1) |
| 8 | Ser-Phe-Asn | 78 | 753 | 0,14, I (4:1) |
| 8a | Phe-Val | 85 | 651 | 0,77, I (4:1) |

a) = MS (DCI, $NH_3$) m/z = $(M+H)^+$
b) = reines unpolares Diastereomer
c) = reines polares Diastereomer

Wie für Beispiel 3 beschrieben, erhielt man durch Kupplung der entsprechenden Säuren mit den Hydrochloriden (Startmaterial) die folgenden Produkte (Tabelle 2)

Tabelle 2

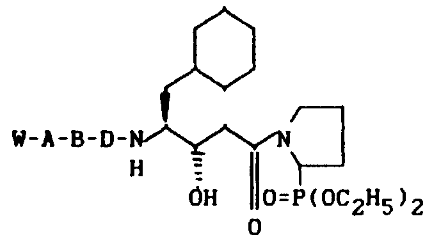

| Beispiel-Nr. | W-A-B-D | Ausbeute (%) | MS (FAB) m/z $(M+H)^+$ | $R_f$/Laufmittel- (Verhältnis) | Startmaterial aus Beispiel |
|---|---|---|---|---|---|
| 9 | Boc-Val | 54 | 604 | 0,36, 0,42, I (93:7) | 2 |
| 10 | Boc-Phe | 62 | 658 | 0,15, 0,24, I (95:5) | 2 |
| 11 | $CH_3(CH_2)_{12}CO$-Phe-Asn | 29 | 876 | 0,21, I (9:1) | 4 |
| 12 | Boc-Phe-Asn | 30 | 766 | 0,30, 1 (9:1) | 4 |
| 13 | Boc-Ser-Phe-Asn | 18 | 853 | 0,27, I (9:1) | 4 |

Beispiel 14

1-{(3S, 4S)-4-[(2S)-3-(tert.Butylsulfonyl)-2-(1-naphthylmethyl)propanoyl]-valinylamino-5-cyclohexyl-3-hydroxypentanoyl}-(2R oder S)-2-(pyrrolidinyl)-phosphonsäurediethylester

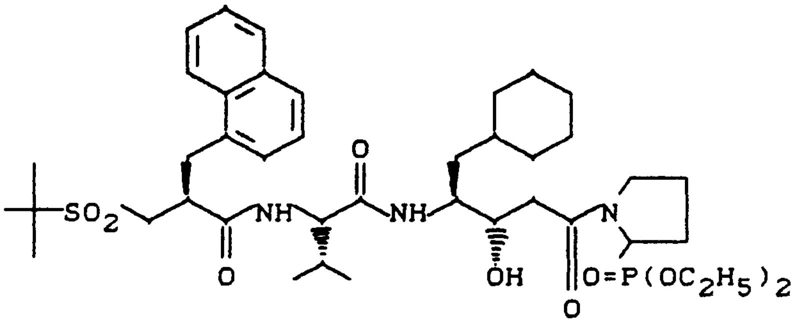

Eine auf 0 °C gekühlte, gerührte Lösung von 159 mg (0,48 mmol) (2S)-3-tert.Butylsulfonyl-2-(1-naphthylmethyl)propionsäure [hergestellt analog H. Bühlmayer et al., J. Med. Chem. 31, 1839 (1988)] und 71 mg (0,53 mmol) HOBT in 5 ml wasserfreiem Dichlormethan wurde mit 104 mg (0,50 mmol) DCC versetzt und 5 min gerührt. Danach wurde eine Lösung von 250 mg (0,43 mmol) der Verbindung aus Beispiel 5 und 0,17 ml (1,5 mmol) N-Methylmorpholin in 5 ml Dichlormethan zugetropft. Das Kühlbad wurde entfernt und die Reaktionsmischung durfte 1 h bei Raumtemperatur rühren. Das Ende der Reaktion wurde dünnschichtchromatographisch festgestellt. Man trennte den entstandenen Harnstoff durch Filtration ab, engte das Filtrat im Vakuum ein und reinigte das Rohprodukt durch Chromatographie an 41 g Kieselgel (Dichlormethan : Methanol 95:5). Man erhielt 223 mg (63% der Theorie) der Titelverbindung als farblosen Schaum.

$R_f$ = 0,39, I (95:5)

MS (FAB): m/z = 820 $(M+H)^+$, 842 $(M+Na)^+$

HPLC: reines Diastereomer

Beispiel 15

1-{(3S, 4S)-4-[(2S)-2-Benzyl-3-(tert.butylsulfonyl)propanoyl]valinylamino]-5-cyclohexyl-3-hydroxypentanoyl}-(2R oder S)-2-(pyrrolidinyl)-phosphonsäurediethylester

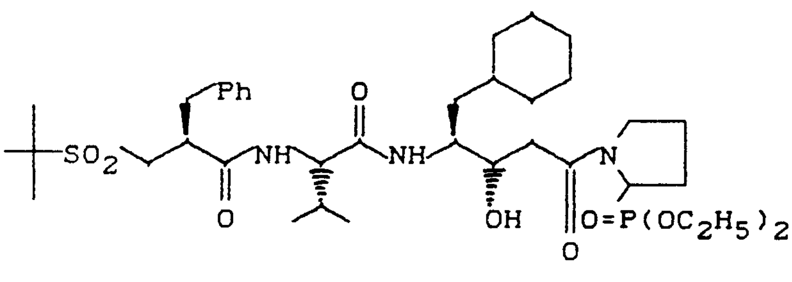

Wie für Beispiel 14 beschrieben erhielt man aus 111 mg (0,39 mmol) (S)-2-Benzyl-3-(tert.butylsulfonyl)-propionsäure [H. Bühlmayer et al., J. Med. Chem. 31, 1839 (1988)] und 205 mg (0,36 mmol) der Verbindung aus Beispiel 5, nach Chromatographie des Rohproduktes an 32 g Kieselgel (I, 95:5) 146 mg (53% der Theorie) der Titelverbindung als farblosen Schaum.

$R_f$ = 0,41, I (95:5)

MS (FAB): m/z = 770 $(M+H)^+$, 792 $(M+Na)^+$

Wie für Beispiel 14 beschrieben, erhielt man durch Kupplung der entsprechenden Säuren mit den Hydrochloriden (Startmaterial) die folgenden Produkte (Tabelle 3):

Tabelle 3

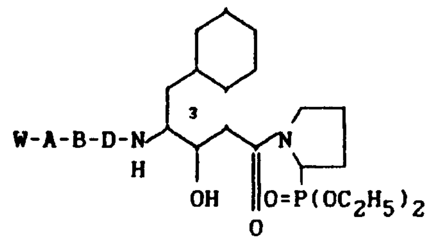

| Beispiel-Nr. | W-A-B-D | 3-OH-Stereochemie | Ausbeute (%) | MS (FAB) m/z $(M+H)^+$ | $R_f$/Laufmittel-(Verhältnis) | Startmaterial aus Beispiel |
|---|---|---|---|---|---|---|
| 15a | Boc-Phe-Val- | S | 56 | 751 | 0,30, I (93:7) | 2 |
| 15b | 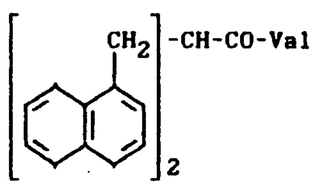 | S | 63 | 827 | 0,24, I (95:5) | 5 |
| 15c | 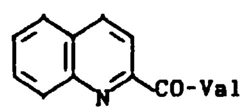 | S | 86 | 659 | 0,08, I (95:5) | 5 |

Fortsetzung Tabelle 3

| Beispiel-Nr. | W-A-B-D | 3-OH-Stereochemie | Ausbeute (%) | MS (FAB) m/z $(M+H)^+$ | $R_f$/Laufmittel-(Verhältnis) | Startmaterial aus Beispiel |
|---|---|---|---|---|---|---|
| 15d | 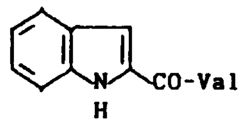 | S | 60 | 647 | 0,05, I (95:5) | 5 |
| 15e | $(CH_3)_3C-CH_2CO$-Phe-Val | S | 57 | 749 | 0,39, i (93:7) | 5 |
| 15f | 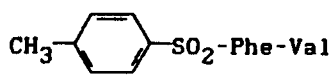 | S | 65 | 805 | 0,21, I (95:5) | 5 |
| 15g | Boc-Ser-Phe-Val | S | 70 | 838 | 0,05, I (93:7) | 5 |
| 15h | Boc-Phe-Gly-Gly | R | 57 | 766 | 0,12, I (9:1) | 29 |
| 15i | 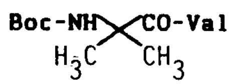 | S | 68 | 689 | 0,10, I (95:5) | 5 |
| 15j | 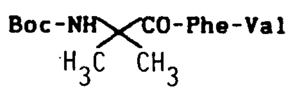 | S | | | | |

Fortsetzung Tabelle 3

| Beispiel-Nr. | W-A-B-D | 3-OH-Stereochemie | Ausbeute (%) | MS (FAB) m/z $(M+H)^+$ | $R_f$/Laufmittel-(Verhältnis) | Startmaterial aus Beispiel |
|---|---|---|---|---|---|---|
| 15k | 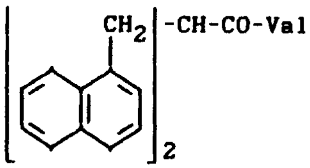 | R | 62 | 826 | 0,21, I (95:5) | 32 |
| 15l | 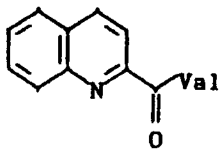 | R | 66 | 859 | 0,13, I (95:5) | 32 |
| 15m | 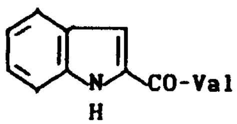 | R | 68 | 647 | 0,06, I (95:5) | 32 |
| 15n | 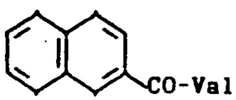 | R | 68 | 658 | 0,11, I (95:5) | 32 |

Beispiel 16

1-{(3S, 4S)-5-Cyclohexyl-3-hydroxy-4-[(1-naphthylaminocarbonyl)phenylalaninyl]aminopentanoyl}-(2R oder S)-2-(pyrrolidinyl)-phosphonsäure-diethylester

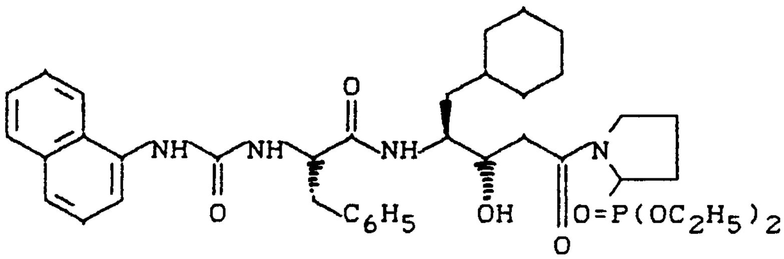

Zu einer gerührten Lösung von 650 mg (1,03 mmol) der Verbindung aus Beispiel 7 und 290 µl (2,07 mmol) Triethylamin in 7 ml wasserfreiem Dichlormethan wurden 160 µl (1,14 mmol) 1-Naphthylisocyanat getropft. Nach 15 min bei Raumtemperatur wurden 5 ml Toluol zugegeben und die Reaktionsmischung wurde im Vakuum eingeengt. Nach Chromatographie des Rückstandes an 40 g Kieselgel (Dichlormethan : Methanol 95:5) erhielt man 530 mg (71% der Theorie) der Titelverbindung als farblosen Hartschaum.
$R_f$ = 0,10 (I, 95:5)
MS (FAB): m/z = 721 $(M+H)^+$, 743 $(M+Na)^+$
HPLC: reines polares Diastereomer

Wie für Beispiel 16 beschrieben erhielt man durch Umsetzung verschiedener Isocyanate mit den entsprechenden Aminen folgende Produkte (Tabelle 4):

Tabelle 4

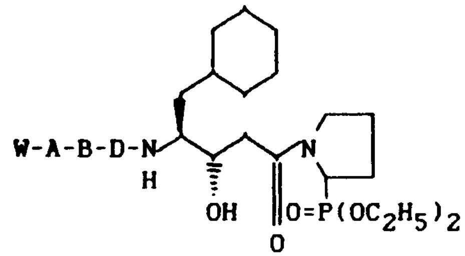

| Beispiel-Nr. | W-A-B-D | Ausbeute (%) | MS (FAB) m/z $(M+H)^+$ | $R_f$/Laufmittel- (Verhältnis) | Startmaterial aus Beispiel |
|---|---|---|---|---|---|
| 17 | 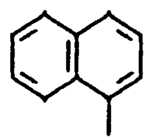 HN-CO-Phe | 57 | 721 | 0,19, I (95:5) | 6[a] |
| 18 | Cl 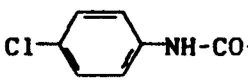 NH-CO-Phe | 67 | 706 | 0,05, I (95:5) | 7 |
| 19 | 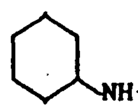 NH-CO-Phe- | 59 | 678 | 0,27, I (93:7) | 7 |
| 20 | $CH_3(CH_2)_{11}$NHCO-Phe- | 83 | 765 | 0,05, I (95:5) | 7 |
| 21 | $(CH_3)_3$C-NH-CO-Phe- | 65 | 651 | 0,08, I (95:5) | 7 |

Fortsetzung Tabelle 4

| Beispiel-Nr. | W-A-B-D | Ausbeute (%) | MS (FAB) m/z $(M+H)^+$ | $R_f$/Laufmittel- (Verhältnis) | Startmaterial aus Beispiel |
|---|---|---|---|---|---|
| 22 | 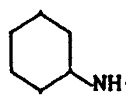 NH-CO-Phe-Val | 34 | 776 | 0,18, 0,20, I (95:5) | 8a |
| 23 | 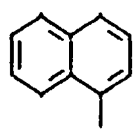 NH-CO-Phe-Val | 37 | 821 | 0,20, 0,25, I (95:5) | 8a |

a) unpolares Diastereomer

Beispiel 24

1-{(3S, 4S)-4-[[(2-Naphthoyl)phenylalaninyl]valinylamino]-5-cyclohexyl-3-hydroxypentanonyl}-(2R,S)-2-(pyrrolidinyl)phosphonsäure-diethylester

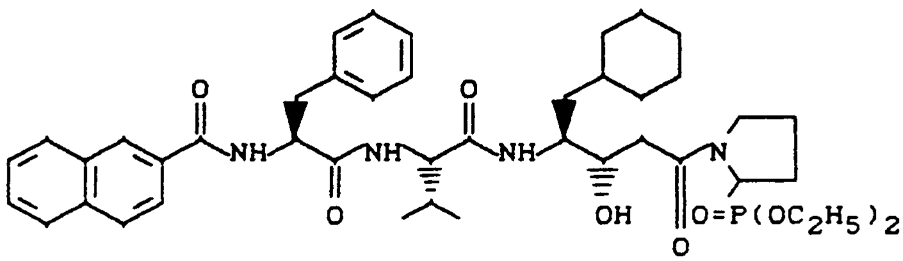

Zu einer gerührten, auf 0° C gekühlten Lösung von 148 mg (0,20 mmol) der Verbindung aus Beispiel 8a und 77 µl (0,7 mmol) N-Methylmorpholin in 1 ml wasserfreiem Dichlormethan wurden 46 mg (0,24 mmol) 2-Naphthoylchlorid getropft. Nach 15 min wurde in ein Gemisch aus 20 ml kalter Natriumbicarbonat-Lösung und 10 ml Ethylacetat gegossen und gut durchgerührt. Die organische Phase wurde abgetrennt, die Wasserphase mit 10 ml Ethylacetat extrahiert und die vereinigten organischen Extrakte über Magnesiumsulfat getrocknet. Nach Abdampfen des Lösemittels im Vakuum und Chromatographie des Rohproduktes an 20 g Kieselgel (Dichlormethan : Methanol 95:5) erhielt man 91 mg (56% der Theorie) der Titelverbindung als farblosen Schaum (Gemisch des polaren und unpolaren Diastereomers).

$R_f$ = 0,13, I (95:5)

MS (FAB): m/z = 806 $(M+H)^+$, 828 $(M+Na)^+$

Wie für Beispiel 24 beschreiben erhielt man durch Acylierung des entsprechenden Hydrochlorids die folgenden Produkte (Tabelle 5):

Tabelle 5

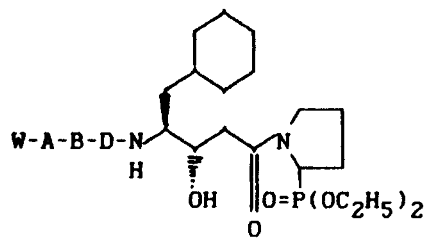

| Beispiel-Nr. | W-A-B-D | Ausbeute (%) | MS (FAB) m/z $(M+H)^+$ | $R_f$/Laufmittel- (Verhältnis) | Startmaterial aus Beispiel |
|---|---|---|---|---|---|
| 25 | $CH_3CO$-Phe-Val | 34 | 693 | 0,13, I (93:7) | 8a |
| 26 | $(CH_3)_3C$-CO-Phe-Val | 46 | 735 | 0,05, I (95:5) | 8a |
| 27 | $CH_3$ 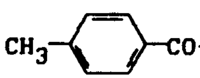 CO-Phe-Val | 55 | 769 | 0,13, I (95:5) | 8a |

Beispiel 28

1-{(3R, 4S)-4-[(tert.Butoxycarbonyl)amino]-5-cyclohexyl-3-hydroxy-pentanoyl]-(2R,S)-2-(pyrrolidinyl)-phosphonsäure-diethylester

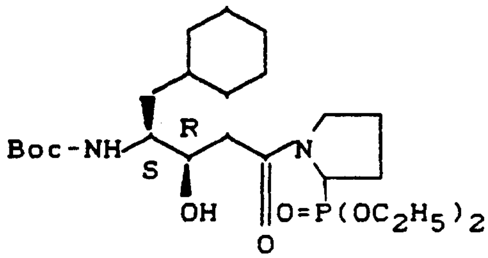

Wie für Beispiel 1 beschrieben erhielt man aus 3,12 g (9,89 mmol) N-(tert.Butoxycarbonyl)-(3R, 4S)-4-amino-5-cyclohexyl-3-hydroxy-pentansäure [Boger et al., J. Med. Chem. 28, 1779 (1985)] und 2,25 g (10,88 mmol) 2(R,S)-2-(Pyrrolidinyl)-phosphonsäurediethylester [US 4 186 268] nach Chromatographie des Rohproduktes an 160 g Kieselgel (Dichlormethan:Methanol 95:5) 3,24 g (65% der Theorie) der Titelverbindung als farblosen Schaum.
$R_f$ = 0,18, I (95:5)
MS (FAB): m/z = 505 $(M+H)^+$, 527 $(M+Na)^+$

Beispiel 29

1-[(3R, 4S)-4-Amino-5-cyclohexyl-3-hydroxypentanoyl]-(2R,S)-2-(pyrrolidinyl)-phosphonsäurediethylester Hydrochlorid

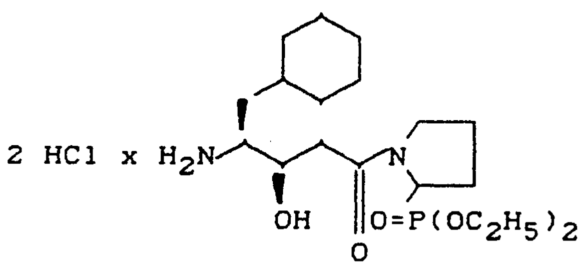

Wie für Beispiel 2 beschrieben erhielt man aus 3,14 g (6,20 mmol) der Verbindung aus Beispiel 29 2,04 g (83% der Theorie) der Titelverbindung als Schaum.
$R_f$ = 0,18, IV
MS (FAB): m/z = 405 $(m+H)^+$

Beispiel 30

1-[(3R, 4S)-4-(tert.Butoxycarbonylvalinyl)amino-5-cyclohexyl-3-hydroxypentanoyl]-(2R,S)-2-(pyrrolidinyl)-phosphonsäurediethylester

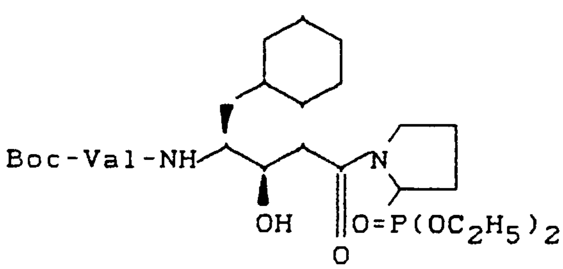

Wie für Beispiel 3 beschrieben erhielt man aus 1,70 g (3,56 mmol) der Verbindung aus Beispiel 29 und

0,85 g (3,92 mmol) tert.Butoxycarbonylvalin nach Chromatographie des Rohproduktes an 250 g Kieselgel (Dichlormethan : Methanol 95:5) 1,387 g (65% der Theorie) der Titelverbindung als farblosen Schaum.
$R_f$ = 0,06, I (95:5)
MS (FAB): m/z = 604 $(M+H)^+$, 626 $(M+Na)^+$.

Beispiel 31

1-[(3R, 4S)-5-Cyclohexyl-3-hydroxy-4-valinylamino-pentanoyl]-(2R,S)-2-(pyrrolidinyl)-phosphonsäurediethylester Hydrochlorid

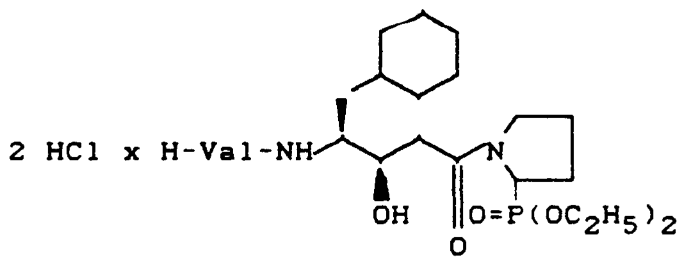

Wie für Beispiel 2 beschrieben erhielt man aus 1,304 g (2,16 mmol) der Verbindung aus Beispiel 31 1,225 g (97% der Theorie) der Titelverbindung als Pulver.
$R_f$ = 0,39, IV
MS (FAB): m/z = 504 $(M+H)^+$

**Patentansprüche**

1. Phosphonopyrrolidin- und piperidinhaltige Pseudopeptide der allgemeine Formel (I)

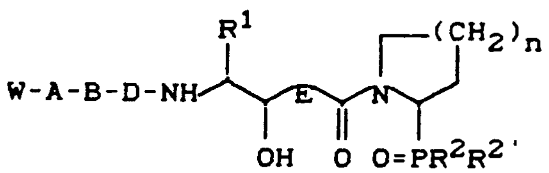

(I)

in welcher
W
- für Wasserstoff oder für eine typische Aminoschutzgruppe steht, oder
- für geradkettiges oder verzweigtes Alkyl oder Alkenyl mit jeweils bis zu 6 Kohlenstoffatomen steht, die gegebenenfalls durch Aryl mit 6 bis 10 Kohlenstoffatomen substituiert sind, oder
- für eine Gruppe der Formel $R^3$-CO-, $R^5R^4$N-CO- oder $R^6$-$SO_2$- steht,

worin

$R^3$
- Wasserstoff, Trifluormethyl oder geradkettiges oder verzweigtes Alkoxy mit bis zu 8 Kohlenstoffatomen oder Alkyl mit bis zu 18 Kohlenstoffatomen bedeutet, das gegebenenfalls bis zu 2-fach durch Aryl mit 6 bis 10 Kohlenstoffatomen oder Pyridyl substituiert ist, oder
- Aryl mit 6 bis 10 Kohlenstoffatomen bedeutet, das gegebenenfalls durch Halogen, Trifluormethyl, Trifluormethoxy oder durch geradkettiges oder verzweigtes Alkyl mit bis zu 8 Kohlenstoffatomen substituiert ist,
- Cycloalkyl mit 3 bis 7 Kohlenstoffatomen bedeutet, oder
- Chinolyl, Indolyl, Pyridyl, Morpholino oder Piperazinyl bedeutet, oder

- einen Rest der Formel

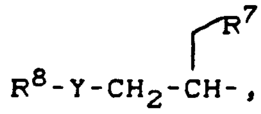, 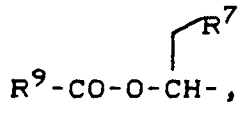, 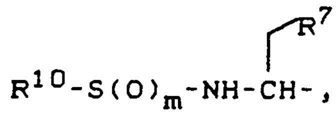,

$T-NH-(CH_2)_p-$ , 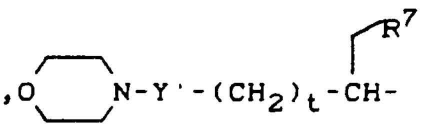

oder 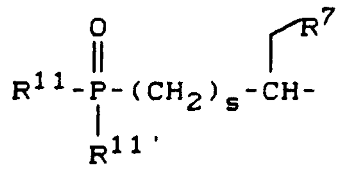

bedeutet,

worin

$R^7$ - Phenyl oder Naphthyl bedeutet,

$R^8$, $R^9$ und $R^{10}$ gleich oder verschieden sind und geradkettiges oder verzweigtes Alkyl mit bis zu 17 Kohlenstoffatomen bedeuten, das gegebenenfalls durch Phenyl oder Naphthyl substituiert ist,

oder Aryl mit 6 bis 10 Kohlenstoffatomen bedeuten, welches seinerseits durch Alkyl mit bis zu 4 Kohlenstoffatomen substituiert ist,

m - eine Zahl 0, 1 oder 2 bedeutet,

T - Morpholino oder Cyclohexyl bedeutet,

p - eine Zahl 1, 2 oder 3 bedeutet,

Y und Y' gleich oder verschieden sind und die CO- oder $SO_2$-Gruppe bedeuten,

t - eine Zahl 0 oder 1 bedeutet,

$R^{11}$ und $R^{11'}$ gleich oder verschieden sind und Hydroxy oder Alkoxy mit bis zu 8 Kohlenstoffatomen bedeuten,

s - eine Zahl 1 oder 2 bedeutet,

$R^4$ und $R^5$ gleich oder verschieden sind und
- Wasserstoff oder
- Aryl mit 6 bis 10 Kohlenstoffatomen bedeuten, das gegebenenfalls durch geradkettiges oder verzweigtes Alkyl mit bis zu 6 Kohlenstoffatomen oder Halogen substituiert ist, oder
- Cycloalkyl mit 3 bis 7 Kohlenstoffatomen bedeuten, oder
- geradkettiges oder verzweigtes Alkyl mit bis zu 18 Kohlenstoffatomen bedeuten,

$R^6$ - geradkettiges oder verzweigtes Alkyl mit bis zu 4 Kohlenstoffatomen oder

Phenyl bedeutet, das seinerseits durch Methyl substituiert sein kann,

A, B und D gleich oder verschieden sind und

- für eine direkte Bindung oder
- für einen Rest der Formel

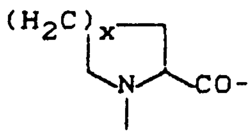

oder

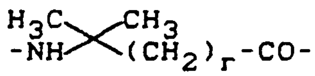

stehen,

worin

x - die Zahl 1 oder 2 bedeutet

und

r - die Zahl 0 oder 1 bedeutet,

oder

- für eine Gruppe der Formel

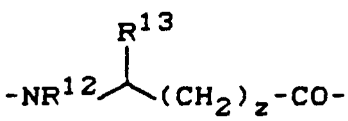

stehen

worin

z - die Zahl 0 oder 1 bedeutet,

$R^{12}$ Wasserstoff oder geradkettiges oder verzweigtes Alkyl mit bis zu 4 Kohlenstoffatomen bedeutet,

$R^{13}$ Wasserstoff, Cycloalkyl mit 3 bis 8 Kohlenstoffatomen oder Aryl mit 6 bis 10 Kohlenstoffatomen bedeutet, oder

- geradkettiges oder verzweigtes Alkyl mit bis zu 8 Kohlenstoffatomen bedeutet, das gegebenenfalls durch Methylthio, Hydroxy, Mercapto, Guanidyl oder durch eine Gruppe der Formel $-NR^{14}R^{15}$ oder $R^{16}$-OC-substituiert ist,

  worin

  $R^{14}$ und $R^{15}$ gleich oder verschieden sind und Wasserstoff, geradkettiges oder verzweigtes Alkyl mit bis zu 8 Kohlenstoffatomen oder Phenyl bedeuten

  und

  $R^{16}$ Hydroxy, Benzyloxy, Alkoxy mit bis zu 6 Kohlenstoffatomen oder die oben aufgeführte Gruppe $-NR^{14}R^{15}$ bedeutet,

oder das gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{14}R^{15}$ substituiert ist,

worin

$R^{14}$ und $R^{15}$ die oben angegebene Bedeutung haben,

oder das gegebenenfalls durch einen 5- oder 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^1$ - für geradkettiges oder verzweigtes Alkyl oder Alkenyl mit bis zu 10 Kohlenstoffatomen steht, die gegebenenfalls durch Cycloalkyl mit 3 bis 8 Kohlenstoffatomen oder Aryl mit 6 bis 10 Kohlenstoffatomen substituiert sind, das seinerseits durch Halogen, Nitro, Hydroxy, Amino oder geradkettiges oder verzweigtes Alkoxy mit bis zu 4 Kohlenstoffatomen substituiert sein kann,

n - für die Zahl 1 oder 2 steht,

$R^2$ und $R^{2'}$ gleich oder verschieden sind und

- für Hydroxy oder Alkoxy mit bis zu 8 Kohlenstoffatomen stehen,

und

E - für einen Rest der Formel $CH_2$, $CF_2$ oder $CH\text{-}OR^{17}$ steht,

worin

$R^{17}$ - Wasserstoff oder geradkettiges oder verzweigtes Alkyl mit bis zu 4 Kohlenstoffatomen bedeutet,

und deren physiologisch unbedenklichen Salze.

**2.** Verbindungen der allgemeinen Formel (I) gemäß Anspruch 1, in welcher

W

- für Wasserstoff, tert.Butyloxycarbonyl (BOC), 9-Fluorenylmethyloxycarbonyl (Fmoc) oder Benzyloxycarbonyl steht, oder
- für geradkettiges oder verzweigtes Alkyl oder Alkenyl mit jeweils bis zu 4 Kohlenstoffatomen steht, die gegebenenfalls durch Phenyl substituiert sind, oder
- für eine Gruppe der Formel $R^3$-CO-, $R^5R^4$N-CO- oder $R^6$-$SO_2$- steht,

worin

$R^3$

- Wasserstoff, Trifluormethyl oder geradkettiges oder verzweigtes Alkoxy mit bis zu 4 Kohlenstoffatomen oder Alkyl mit bis zu 16 Kohlenstoffatomen bedeutet, das gegebenenfalls bis zu 2-fach durch Phenyl, Naphthyl oder Pyridyl substituiert ist, oder
- Phenyl oder Naphthyl bedeutet, die gegebenenfalls durch Fluor, Chlor, Trifluormethyl, Trifluormethoxy oder durch geradkettiges oder verzweigtes Alkyl mit bis zu 6 Kohlenstoffatomen substituiert sind,
- Cyclopropyl, Cyclopentyl, Cyclohexyl, Chinolyl, Indolyl, Pyridyl, Morpholino oder Piperazinyl bedeutet, oder
- einen Rest der Formel

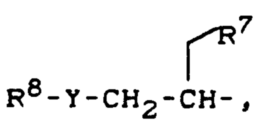, 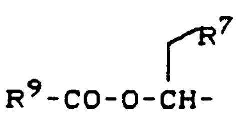 oder

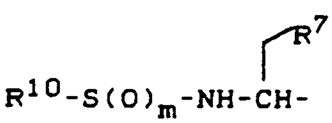

bedeutet,

worin

Y - die CO- oder $SO_2$-Gruppe bedeutet,

$R^7$ - Phenyl oder Naphthyl bedeutet,

$R^8$, $R^9$ und $R^{10}$ gleich oder verschieden sind und geradkettiges oder verzweigtes Alkyl mit bis zu 15 Kohlenstoffatomen, Tolyl, Phenyl oder Naphthyl bedeuten,

m - eine Zahl 1 oder 2 bedeutet,

$R^4$ und $R^5$ gleich oder verschieden sind und
- Wasserstoff oder
- Phenyl oder Naphthyl bedeuten, die gegebenenfalls durch geradkettiges oder verzweigtes Alkyl mit bis zu 4 Kohlenstoffatomen, Fluor oder Chlor substituiert sind,
- Cyclopropyl, Cyclopentyl oder Cyclohexyl bedeuten, oder
- geradkettiges oder verzweigtes Alkyl mit bis zu 16 Kohlenstoffatomen bedeuten,

$R^6$ - Phenyl bedeutet, das durch Methyl substituiert ist,

A, B und D gleich oder verschieden sind und
- für eine direkte Bindung oder
- für Prolin stehen, oder
- für einen Rest der Formel

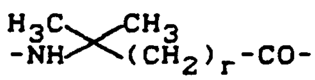

stehen,

worin

r die Zahl 0 oder 1 bedeutet

- für eine Gruppe der Formel

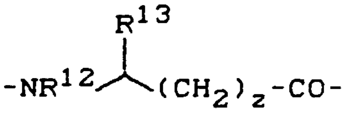

stehen

worin

z - die Zahl 0 oder 1 bedeutet,

$R^{12}$- Wasserstoff, Methyl oder Ethyl bedeutet,

$R^{13}$
- Wasserstoff, Cyclopentyl, Cyclohexyl oder Phenyl bedeutet,
- oder geradkettiges oder verzweigtes Alkyl mit bis zu 6 Kohlenstoffatomen

bedeutet, das gegebenenfalls durch Methylthio, Hydroxy, Mercapto, Guanidyl, Amino, Carboxy oder $H_2N$-CO-substituiert sein kann,

oder durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,

oder durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^1$ - für geradkettiges oder verzweigtes Alkyl oder Alkenyl mit bis zu 8 Kohlenstoffatomen steht, die gegebenenfalls durch Cyclopropyl, Cyclobutyl, Cyclopentyl, Cyclohexyl, Cycloheptyl oder Phenyl substituiert sind, das seinerseits durch Fluor, Chlor, Brom, Nitro, Hydroxy oder Amino substituiert ist,

n - für die Zahl 1 oder 2 steht,

$R^2$ und $R^{2'}$ gleich oder verschieden sind und

- für Hydroxy oder Alkoxy mit bis zu 6 Kohlenstoffatomen stehen,

und

E - für einen Rest der Formel

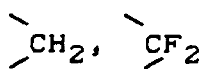 oder

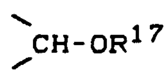

steht,

worin

$R^{17}$ - Wasserstoff oder geradkettiges oder verzweigtes Alkyl mit bis zu 4 Kohlenstoffatomen steht

und deren physiologisch unbedenklichen Salze.

**3.** Verbindungen der allgemeinen Formel (1) gemäß Anspruch 1, in welcher

W

- für Wasserstoff, tert.Butyloxycarbonyl (BOC) oder Benzyloycarbonyl steht, oder
- für Allyl oder Benzyl steht,
- für eine Gruppe der Formel $R^3$-CO-, $R^5R^4N$-CO-oder $R^6$-$SO_2$- steht,

worin

$R^3$

- Wasserstoff, Trifluormethyl oder geradkettiges oder verzweigtes Alkyl mit bis zu 14 Kohlenstoffatomen bedeutet, das gegebenenfalls bis zu 2-fach durch Phenyl, Naphthyl oder Pyridyl substituiert ist, oder
- Phenyl oder Naphthyl bedeutet, die gegebenenfalls durch Fluor, Chlor, Trifluormethyl, Trifluormethoxy oder durch geradkettiges oder verzweigtes Alkyl mit bis zu 4 Kohlenstoffatomen substituiert sind,
- Cyclopropyl, Cyclopentyl, Cyclohexyl, Chinolyl, Indolyl, Pyridyl, Morpholino oder Piperazinyl bedeutet, oder
- einen Rest der Formel

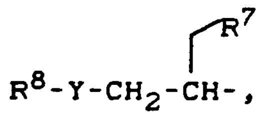 oder

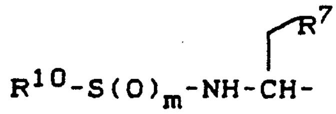

bedeutet,

worin

Y -die CO- oder $SO_2$-Gruppe bedeutet,

$R^7$ -Phenyl oder Naphthyl bedeutet,

$R^8$ und $R^{10}$ gleich oder verschieden sind und geradkettiges oder verzweigtes Alkyl mit bis zu 14 Kohlenstoffatomen, Tolyl, Phenyl oder Naphthyl bedeuten,

m -eine Zahl 1 oder 2 bedeutet,

$R^4$ und $R^5$ gleich oder verschieden sind und

- Wasserstoff oder
- Phenyl oder Naphthyl bedeuten, die gegebenenfalls durch Methyl, Fluor oder Chlor substituiert sind,
- Cyclopropyl, Cyclopentyl oder Cyclohexyl bedeuten, oder
- geradkettiges oder verzweigtes Alkyl mit bis zu 14 Kohlenstoffatomen bedeuten,

$R^6$ - Phenyl bedeutet, das durch Methyl substituiert ist,

A, B und D gleich oder verschieden sind und

- für eine direkte Bindung stehen, oder
- für Prolin stehen, oder
- für einen Rest der Formel

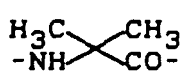

stehen,

- für eine Gruppe der Formel

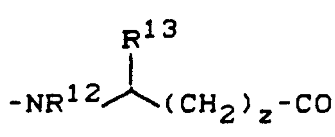

stehen,

worin

z - die Zahl 0 oder 1 bedeutet,

$R^{12}$- Wasserstoff oder Methyl bedeutet,

$R^{13}$
- Wasserstoff, Cyclopentyl oder Cyclohexyl bedeutet,
- oder geradkettiges oder verzweigtes Alkyl mit bis zu 4 Kohlenstoffatomen bedeutet, das gegebenenfalls durch Methylthio, Hydroxy, Mercapto, Guanidyl, Amino, Carboxy oder $H_2N$-CO-substituiert ist,

  oder durch Cyclohexyl, Naphthyl oder Phenyl substituiert ist, das seinerseits durch Hydroxy, Fluor, Chlor oder Alkoxy mit bis zu 4 Kohlenstoffatomen substituiert sein kann, oder

  durch Indolyl, Imidazolyl, Triazolyl, Pyridyl der Pyrazolyl substituiert ist, wobei die NH-Funktion gegebenenfalls durch Methyl, Benzyloxymethylen oder t-Butyloxycarbonyl (Boc) geschützt sind,

$R^1$ - für geradkettiges oder verzweigtes Alkyl mit bis zu 6 Kohlenstoffatomen steht, das gegebenenfalls durch Cyclopropyl, Cyclopentyl, Cyclohexyl oder Phenyl substituiert ist, das seinerseits durch Hydroxy substituiert sein kann,

n - für die Zahl 1 oder 2 steht,

$R^2$ und $R^{2'}$ gleich oder verschieden sind und
- für Hydroxy oder Alkoxy mit bis zu 4 Kohlenstoffatomen stehen,

und

E -für die

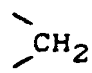

oder die

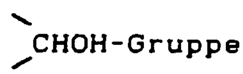

steht

und deren physiologisch unbedenklichen Salze.

**4.** Verfahren zur Herstellung der Verbindungen der allgemeinen Formel (I)

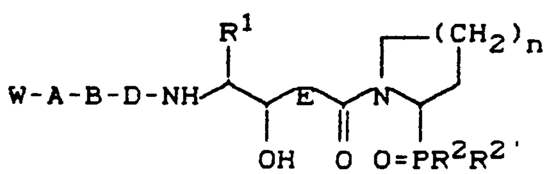

(I)

in welcher

W, A, B, D, $R^1$, $R^2$, $R^{2'}$, E und n die oben angegebene Bedeutung haben,

dadurch gekennzeichnet, daß man

[A] Verbindungen der allgemeinen Formel (Ia)

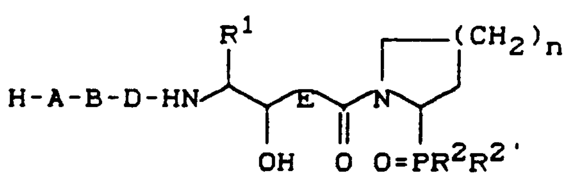

(Ia)

in welcher

A, B, D, $R^1$, $R^2$, $R^{2'}$, E und n die oben angegebene Bedeutung haben,

mit Verbindungen der allgemeinen Formeln (II) oder (III)

W-X (II)

$(W')_2O$ (III)

in welcher

W die oben angegebene Bedeutung hat

X für Halogen, vorzugsweise für Chlor steht,

und

W' für die Gruppe $CF_3CO$ oder $CH_3CO$ steht,

nach den in der Peptidchemie üblichen Bedingungen, in inerten Lösemitteln, in Anwesenheit einer Base umsetzt,

oder
[B] entweder Verbindungen der allgemeinen Formel (Ib)

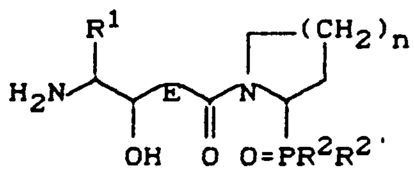

(Ib)

in welcher

$R^1$, $R^2$, $R^{2'}$, E und n die oben angegebene Bedeutung haben,

direkt mit Verbindungen der allgemeinen Formel (IV)

W-A'-B'-D'-OH (IV)

in welcher

W die oben angegebene Bedeutung hat,

A', B' oder D' die oben angegebene Bedeutung von A, B oder D haben und nicht gleichzeitig für eine Bindung stehen,

oder Verbindungen der allgemeinen Formel (Ic)

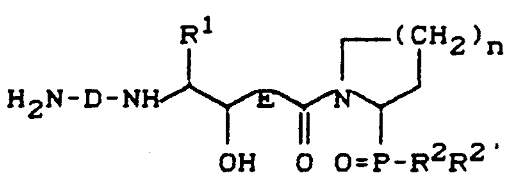

(Ic)

in welcher

D, $R^1$, $R^2$, $R^{2'}$, E und n die oben angegebene Bedeutung haben,

mit Verbindungen der allgemeinen Formel (IVa)

W-A'-B'-OH (IVa)

in welcher

W, A' und B' die oben angegebene Bedeutung haben,

unter Aktivierung der Carbonsäure, in inerten Lösemitteln, gegebenenfalls in Anwesenheit einer Base und eines Hilfsstoffes umsetzt,

oder
[C] Verbindungen der allgemeinen Formeln (V) und (VI)

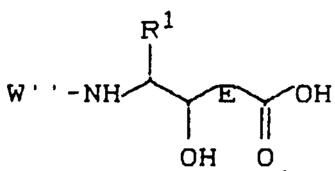

(V)

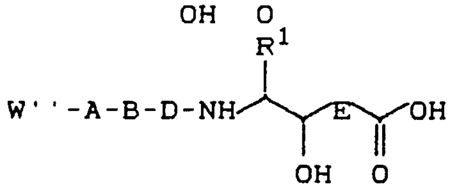

(VI)

in welcher

$R^1$, W, A, B, D und E die oben angegebene Bedeutung haben

und

W'' für eine Aminoschutzgruppe, vorzugsweise für BOC steht,

mit Verbindungen der allgemeinen Formel (VII)

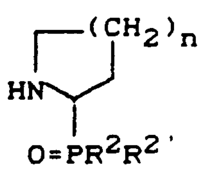

(VII)

in welcher

$R^2$, $R^{2'}$ und n die oben angegebene Bedeutung haben,

unter Aktivierung der Carbonsäuren, gegebenenfalls in Anwesenheit einer Base und eines Hilfsstoffes kondensiert und im Fall der Verbindungen der allgemeinen Formel (V) die Schutzgruppe W'' anschließend nach üblicher Methode abspaltet und gegebenenfalls mit den Verbindungen der allgemeinen Formeln (IV) oder (IVa) nach der unter Verfahren [B] beschriebenen Methode weiter umsetzt.

**5.** Arzneimittel enthaltend eine oder mehrere Verbindungen der Ansprüche 1, 2 oder 3.

**6.** Antivirale Mittel enthaltend eine oder mehrere Verbindungen der Ansprüche 1, 2 oder 3.

**7.** Verwendung der Verbindungen der Ansprüche 1, 2 oder 3 zur Herstellung von antiviralen Arzneimittel.